# DEMANDE DE BREVET EUROPEEN

(11) **EP 2 277 593 A1**
(43) Date de publication de la demande: **26.01.2011**
(21) Numéro de dépôt: 09166268.4
(22) Date de dépôt: 23.07.2009
(51) Int. Cl.: A61N 5/10, A61B 8/00, A61B 8/08

(54) **Dispositif de pilotage d'une cible de faisceau sur une zone in-vivo**

(71) Demandeur: Siemens Schweiz AG, 8047 Zürich (CH)
(72) Inventeur: Cloutot, Laurent, 8956, Killwangen (CH)
(74) Mandataire: Fischer, Michael

(57) **Abrégé**

La présente invention décrit un dispositif de pilotage d'une cible de faisceau (BEAM) sur une zone limitée (C), la dite zone étant une zone in-vivo d'un sujet, comprenant :
- au moins une sonde à ultrason (E) apte à visualiser et localiser spatialement la zone limitée (C),
- au moins une partie émettrice/réceptrice de la sonde est maintenue au moyen d'un support mobile (SE) dans une ouverture d'un plan (T) contre lequel le sujet est superficiellement appuyé, le dit support étant orientable par rapport au plan au moyen d'un premier module de commande (COM1) régi par une unité de contrôle (CTRL),
- un deuxième module de commande (COM2) pilote le point cible sous au moins un mode d'asservissement spatial à la première commande (COM1) afin de faire converger la cible vers la zone localisée par la sonde.

## Description

Dispositif de pilotage d'une cible de faisceau sur une zone in-vivo

La présente invention concerne un dispositif de pilotage d'une cible de faisceau sur une zone in-vivo selon le préambule de la revendication 1.

Actuellement, un objectif majeur réside dans la visualisation de parties internes telles qu'in-vivo chez un sujet de type humain ou animal ou un mannequin dit aussi « fantôme ». En particulier, cet objectif est crucial dès lors qu'une intervention telle qu'une irradiation locale radiothérapeutique ou technologiquement équivalente en dépend afin de pouvoir localement irradier une zone limitée incluant des cellules à détruire tout en préservant de l'irradiation de tissus et cellules saines avoisinés aux cellules malsaines.

Actuellement par exemple, lors d'une séance de radiothérapie sur des cellules cancéreuses telle qu'une tumeur ou un carcinome, un dispositif annexe à l'installation de radiothérapie permet de prendre des prises de vue de type radiographique afin de visualiser et localiser ladite tumeur in-vivo dans le référentiel de l'installation de radiothérapie, ceci aux fins de contrôler que le faisceau irradiant concentre bien son énergie sur la tumeur. Ce procédé à base d'irradiation nuisible (rayons X) n'est toutefois pas sans danger pour un patient déjà affaibli par un cancer et ne permet pas une visualisation ainsi qu'une localisation d'une cible à intervalles fréquents et donc a fortiori en temps réel lors de séances d'irradiation. De tels procédés sont aussi volumineux, coûteux et nécessitent de multiples interventions humaines pour leur positionnement, leur fonctionnement et leur maintenance. Il en ressort que le pilotage d'une cible de faisceau irradiant sur une zone in-vivo lors d'un diagnostic, d'un planning ou d'une thérapie se révèle encore trop statique et donc imprécis au cas où la zone in-vivo à cibler se déplace, par exemple simplement du fait de la respiration d'un sujet.

Un but de la présente invention est ainsi de pouvoir localiser un volume de cellules in-vivo, telles que des cellules cancéreuses, afin de permettre un pilotage spatial dynamique d'une cible (virtuelle ou non) de faisceau sur une zone in-vivo, en vue par exemple du bon planning ou suivi d'une potentielle séance d'irradiation desdites cellules tout en épargnant précisément des tissus sains périphériques.

Enfin, un problème alternatif de l'invention concerne le fait que la visualisation et la localisation d'une tumeur par exemple doit être effectuée en préservant la santé mais aussi le confort et la quiétude du patient afin de lui éviter tout stress complémentaire à un potentiel diagnostic voire une thérapie plus lourde et astreignante. Ainsi, toute irradiation invasive ou du moins nuisible, en supplément à celle liée à une possible radiothérapie ou une thérapie similaire, ainsi que l'usage de méthodes de maintien du patient, de marquage ou autre contrainte infligée au patient doit être minimisé, et idéalement éliminé.

L'invention propose ainsi un dispositif approprié à résoudre cet ensemble de problèmes et décrit au travers de la revendication 1.

Ainsi, l'invention prévoit un dispositif de pilotage spatial d'une cible de faisceau sur une zone limitée, la dite zone étant une zone in-vivo d'un sujet, ledit faisceau pouvant être en particulier apte à simuler, initier ou continuer une irradiation subséquente sur au moins une partie de la zone. Le présent dispositif comprend principalement :
- au moins une sonde à ultrason apte à visualiser et localiser spatialement la zone limitée,
- au moins une partie émettrice/réceptrice de la sonde qui est maintenue au moyen d'un support mobile dans une ouverture d'un plan contre lequel le sujet est superficiellement appuyé, le dit support étant déplaçable ou/et orientable par rapport au plan au moyen d'un premier module de commande régi par une unité de contrôle,
- un deuxième module de commande qui pilote le point cible sous au moins un mode d'asservissement spatial à la première commande afin de faire converger la cible vers la zone localisée par la sonde.

En raison de l'intégration avantageuse d'une sonde à ultrasons, il est ainsi possible de visualiser et localiser la zone limitée (volume de cellules tumorales par exemple) et donc de piloter la cible d'un faisceau annexe sur ladite zone de façon continue et en temps réel, même chez un patient lourdement malade et affaibli, car les ultrasons n'ont pas d'effets nocifs et affaiblissants connus. Si le patient bouge par rapport à une table où il est allongé, le support mobile initialement disposé pour permettre d'inclure la zone visée et son voisinage dans son champ de vision (eux-mêmes en possible mouvement, par exemple par de simples raisons respiratoire, de flux sanguin ou d'autres aspects dynamiques in-vivo) assure un déplacement libre ou forcé de la sonde afin de toujours cadrer la zone visée dans son champ de vision. Cette unique prédisposition de la sonde peut-être réglée en début d'une phase de visualisation grâce à un opérateur, afin d'assurer une visualisation et localisation sans faille a posteriori pour finalement asservir le déplacement de la cible de faisceau. Il est à noter que le faisceau peut être inactivé provisoirement lors de déplacement ou de réglage divers de la position de la sonde.

Les sondes à ultrasons actuelles permettent une vision bi- ou tridimensionnelle et donc, une fois que la zone limitée (tumeur) est localisée, il est possible de recalculer sa position par la simple connaissance du déplacement de la sonde dans son support mobile par rapport au plan d'appui du patient (fixe ou du moins spatialement connu dans le référentiel de l'installation annexe de diagnostic/thérapie). Ladite localisation de la zone ciblée et donc le pilotage de la cible de faisceau devant être cadrée sur ladite zone ciblée est donc fort exacte spatialement et absolument dynamique temporellement.

Un ensemble de sous-revendications présente également des avantages de l'invention sous plusieurs aspects.

Un exemple de réalisation du dispositif selon l'invention est fourni à l'aide de la figure suivante :

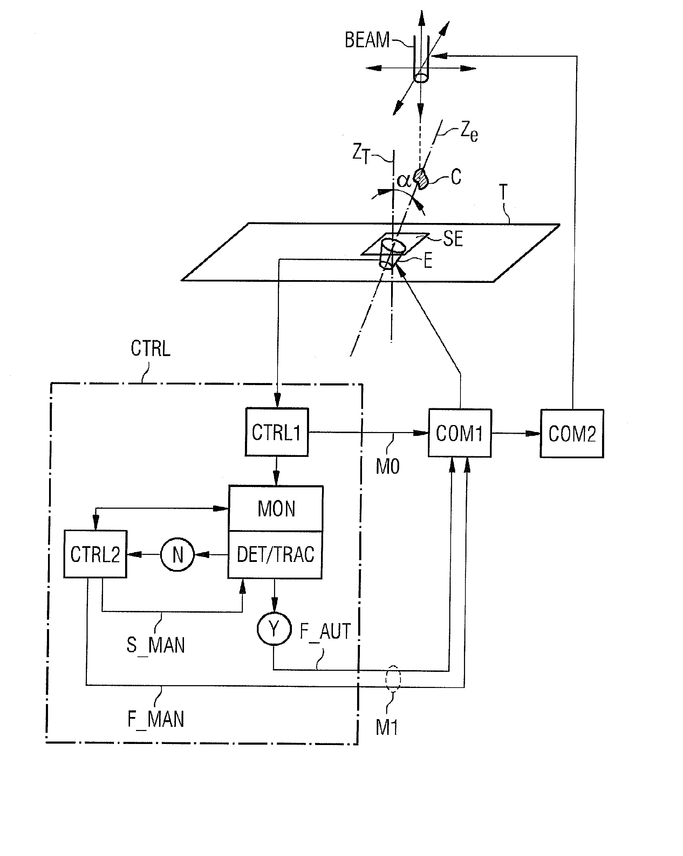

Figure 1 Schéma d'un dispositif de pilotage selon l'invention.

**Figure 1** présente un schéma d'un dispositif de pilotage spatial et dynamique d'une cible de faisceau BEAM sur une zone limitée C, la dite zone étant une zone in-vivo tel qu'un ensemble de cellules malsaines d'un sujet tel qu'un humain (ici non représentée, mais qui serait allongé sur une table ou du moins partiellement appuyé contre un plan T).

Ledit dispositif de pilotage comprend les caractéristiques suivantes :
- au moins une sonde à ultrason E apte à visualiser et localiser spatialement la zone limitée C,
- au moins une partie émettrice/réceptrice de la sonde est positionnable et orientable, idéalement en étant au moins maintenue au moyen d'un support mobile SE dans une ouverture du plan T contre lequel le sujet est superficiellement appuyé, le dit support étant ainsi orientable par rapport au plan au moyen d'un premier module de commande COM1 régi par une unité de contrôle CTRL,
- un deuxième module de commande COM2 pilote spatialement le point cible sous au moins un mode d'asservissement de type spatial à la première commande COM1 afin de faire converger spatialement la cible vers la zone localisée par la sonde et au mieux de synchroniser en permanence la superposition exacte leur deux positions librement variables selon les déplacement de ladite zone.

La figure 1 montre ainsi que l'axe directeur principal Z_{E} du champ de vision de la sonde à ultrasons E est légèrement incliné par rapport à un axe d'incidence Z_{T} du plan T qui ici correspond à l'axe principal d'un faisceau annexe de type radio-thérapeutique par exemple. De cette façon, il est possible de maintenir la sonde écartée et donc protégée de toute altération et interférence provenant du faisceau à haute énergie dont la cible doit se focaliser sur la zone limitée C. D'autres dispositions relatives entre la sonde et le faisceau peuvent être employées à ces mêmes fins, c'est-à-dire pour éviter de faire coïncider les axes principaux de la sonde et du faisceau. A cet effet, le dispositif selon l'invention prévoit que le premier et le deuxième module de commande COM1, COM2 coopèrent mutuellement afin d'assurer que la sonde soit en permanence écarté du faisceau.

Plus précisément, le dispositif selon l'invention prévoit que l'unité de contrôle CTRL comprend un module de visualisation MON de la zone C, ledit module intégrant un moyen de sélection spatiale Y de ladite zone, laquelle permettant à au moins un opérateur CTRL2 ou/et un algorithme de détection et traçage spatial DET/TRAC de la zone C de piloter l'orientation de la sonde afin de conserver la zone sélectionnée dans un champ spatial défini des ultrasons émis par ladite sonde.

En particulier, l'unité de contrôle CTRL délivre deux signaux F_MAN, F_AUT de sortie M1 au premier module de commande COM1 permettant d'assurer deux modes d'asservissement Y, N de sorte que si, sous un mode d'asservissement automatique Y, l'algorithme de détection et traçage DET/TRAC de la zone C présente une irrégularité, le mode d'asservissement automatique Y est commutable sur un mode d'asservissement manuel N régi par l'unité de contrôle manuel CTRL2 du positionnement de la sonde E. Ainsi, un pilotage automatique peut être assisté manuellement pour éviter toute erreur due à un quelconque artefact dans la prise de vue de la sonde ou la détection et reconnaissance de la zone visée/ciblée.

Egalement, sous le mode d'asservissement manuel N, l'unité de contrôle manuel CTRL2 du positionnement de la sonde E est aussi potentiellement désactivable et réactive alors un mode d'asservissement automatique Y au moyen d'un signal sécuritaire S_MAN délivré à une entrée d'unité d'exécution de l'algorithme de détection et traçage DET/TRAC.

Le dispositif selon l'invention peut aussi prévoir que l'unité de contrôle CTRL, CTRL1 est commutable, à partir ou en addition au mode d'asservissement manuel N, sur un mode de commande d'orientation libre M0 du support, en ce que la sonde est librement auto-orientable selon des directions de poussée mécanique exercée par appui d'une partie superficielle du sujet (= par exemple dos du patient) sur la sonde. Suivant ce schéma, le mode d'asservissement à orientation libre peut aussi être assisté le cas échéant par une commutation provisoire sur le mode d'asservissement manuel N (= mode forcé par un opérateur) ou par une assistance de pilotage sous le mode d'asservissement de type automatique Y.

Afin de permettre un pilotage asservi de la cible plus précis, le champ spatial des ultrasons est réglable au moyen de l'unité de contrôle CTRL et du premier module de commande COM1, en particulier en fonction de la distance mesurée entre la zone C et la sonde.

Afin de pouvoir localiser la zone ciblée dans le référentiel même de l'installation contrôlant la cible de faisceau, l'unité de contrôle CTRL délivre un jeu de coordonnées spatiales de la zone in-vivo limitée dans un premier référentiel lié à la sonde, et des moyens de détermination de l'orientation/position instantanée de la sonde délivrent ledit jeu de coordonnées dans un deuxième référentiel lié au plan T, lui-même a posteriori lié à un troisième référentiel lié à la cible (ou son installation de contrôle de position).

Au moins un film, un volume ou une enceinte de gel acoustique est aussi disposé entre la sonde et une partie superficielle du sujet afin d'évacuer tout interstice d'air sur un canal d'ultrasons entre la sonde et la dite partie superficielle d'appui du sujet. Ainsi, des artefacts de mesure de la sonde peuvent être évités. Cela permet aussi une surface de contact plus souple et donc confortable entre la sonde et le sujet.

En particulier pour pouvoir être prédisposé dans une région d'intérêt comprenant la zone ciblée, l'ouverture est positionnable par entrainement du support mobile sur au moins une dimension du plan ou de la table. De plus, le support mobile SE est inclinable autour d'une normale Z_{T} au plan T permettant ainsi un balayage visuel d'axe principal dans un volume conique.

Le support mobile SE peut comprendre des moyens de détermination de sa position et orientation dans un repère tridimensionnel lié au plan, ladite position étant communiquée instantanément à l'unité de contrôle CTRL.

Enfin, le dispositif de pilotage selon l'invention est ainsi adapté pour toute cible comprenant un ensemble de points ayant une étendue adaptable aux contours d'un volume de la zone in-vivo instantanément visualisée, ledit volume comprenant des cellules organiques à caractère malsain pour le sujet, tel qu'une tumeur ou un carcinome. La cible peut donc être ponctuelle, surfacique ou volumétrique en fonction de la zone visée.

## Revendications

1. Dispositif de pilotage d'une cible de faisceau (BEAM) sur une zone limitée (C), la dite zone étant une zone in-vivo d'un sujet, comprenant :
- au moins une sonde à ultrason (E) apte à visualiser et localiser spatialement la zone limitée (C),
- au moins une partie émettrice/réceptrice de la sonde est maintenue au moyen d'un support mobile (SE) dans une ouverture d'un plan (T) contre lequel le sujet est superficiellement appuyé, le dit support étant déplaçable et orientable par rapport au plan au moyen d'un premier module de commande (COM1) régi par une unité de contrôle (CTRL),
- un deuxième module de commande (COM2) pilote le point cible sous au moins un mode d'asservissement spatial à la première commande (COM1) afin de faire converger la cible vers la zone localisée par la sonde.

2. Dispositif selon revendication 1, pour lequel l'unité de contrôle (CTRL) comprend un module de visualisation (MON) de la zone (C), ledit module intégrant un moyen de sélection spatiale (Y) de ladite zone, laquelle permettant à au moins un opérateur (CTRL2) ou/et un algorithme de détection et traçage (DET/TRAC) de la zone (C) de piloter l'orientation de la sonde afin de conserver la zone sélectionnée dans un champ spatial défini des ultrasons émis par ladite sonde.

3. Dispositif selon revendication 2, pour lequel l'unité de contrôle (CTRL) délivre deux signaux de sortie (F_MAN, F_AUT, M1) au premier module de commande (COM1) permettant deux modes d'asservissement (Y, N) de sorte que si, sous un mode d'asservissement automatique (Y), l'algorithme de détection et traçage (DET/TRAC) de la zone (C) présente une irrégularité, le mode d'asservissement automatique (Y) est commutable sur un mode d'asservissement manuel (N) régi par une unité de contrôle manuel (CTRL2) du positionnement de la sonde (E).

4. Dispositif selon revendication 3, pour lequel sous le mode d'asservissement manuel (N), l'unité de contrôle manuel (CTRL2) du positionnement de la sonde (E) est désactivable et réactive un mode d'asservissement automatique (Y) au moyen d'un signal sécuritaire (S MAN).

5. Dispositif selon une des revendications précédentes, pour lequel l'unité de contrôle (CTRL) est commutable (N) sur un mode de commande d'orientation libre (M0) du support, en ce que la sonde est librement auto-orientable selon des directions de poussée mécanique exercée par appui d'une partie superficielle du sujet sur la sonde.

6. Dispositif selon une des revendications précédentes, pour lequel le champ spatial des ultrasons est réglable au moyen de l'unité de contrôle (CTRL) et du premier module de commande (COM1), en particulier en fonction de la distance de la zone (C) et la sonde.

7. Dispositif selon une des revendications précédentes, pour lequel l'unité de contrôle (CTRL) délivre un jeu de coordonnées spatiales de la zone dans un premier référentiel lié à la sonde, et des moyens de détermination de l'orientation instantanée de la sonde délivrent ledit jeu de coordonnées dans un deuxième référentiel lié au plan (T) et a posteriori lié à un troisième référentiel lié à la cible.

8. Dispositif selon une des revendications précédentes, pour lequel au moins un film, un volume ou une enceinte de gel acoustique est disposé entre la sonde et une partie superficielle du sujet afin d'évacuer un interstice d'air sur un canal d'ultrasons entre la sonde et la dite partie superficielle.

9. Dispositif selon une des revendications précédentes, pour lequel par entrainement du support mobile, l'ouverture est positionnable sur au moins une dimension du plan.

10. Dispositif selon une des revendications précédentes, pour lequel le support mobile (SE) est inclinable autour d'une normale (Z_{T}) au plan (T).

11. Dispositif selon une des revendications précédentes, pour lequel le support mobile (SE) comprend des moyens de détermination de sa position dans un repère tridimensionnel lié au plan, ladite position étant communiquée instantanément à l'unité de contrôle (CTRL).

12. Dispositif selon une des revendications précédentes, pour lequel la cible est un ensemble de points ayant une étendue adaptable aux contours d'un volume de la zone in-vivo instantanément visualisée, ledit volume comprenant des cellules organiques à caractère malsain pour le sujet, tel qu'une tumeur ou un carcinome.

13. Dispositif selon une des revendications précédentes, pour lequel le premier et le deuxième module de commande (COM1, COM2) coopèrent mutuellement afin d'assurer que la sonde soit en permanence écarté du faisceau.
